# EUROPEAN PATENT SPECIFICATION

(11) **EP 1 229 922 B1**
(45) Date of publication and mention of the grant of the patent: **06.06.2007**
(21) Application number: 00976137.0
(22) Date of filing: 15.11.2000
(51) Int. Cl.: A61K 35/56, A61K 38/17, A61K 31/4015, A61P 35/00

(54) **APLIDINE TREATMENT OF CANCERS**
BEHANDLUNG VON KREBSERKRANKUNGEN DURCH APLIDIN
TRAITEMENT A L'APLIDINE DE CANCERS

(30) Priority: 15.11.1999 GB 9927006; 09.03.2000 GB 0005701; 29.03.2000 GB 0007639; 23.06.2000 GB 0015496; 13.10.2000 GB 0025209
(43) Date of publication of application: 14.08.2002
(60) Divisional application: 06022134.8
(73) Proprietor: PHARMA MAR, S.A., 28760 Tres Cantos, Madrid (ES)
(72) Inventor: FAIRCLOTH, Glynn Thomas, Pharma Mar USA Inc., Cambridge, MA 02139-4616 (US); TWELVES, Chris, University of Glasgow, Bearsden, Glasgow G61 1BD (GB); PAZ-ARES, Luis, E-28015 Madrid (ES)
(74) Representative: Ruffles, Graham Keith
(86) International application number: PCT/GB2000/004349
(87) International publication number: WO 2001/035974

(56) References cited:
- EP-A- 0 048 149
- WO-A-91/04985
- US-A- 6 156 724
- FAIRCLOTH, G. (1) ET AL: "Schedule-dependency of aplidine, a marine depsipeptide with antitumor activity." PROCEEDINGS OF THE AMERICAN ASSOCIATION FOR CANCER RESEARCH ANNUAL MEETING, (MARCH, 1999) VOL. 40, PP. 394-395. MEETING INFO.: 90TH ANNUAL MEETING OF THE AMERICAN ASSOCIATION FOR CANCER RESEARCH PHILADELPHIA, PENNSYLVANIA, USA APRIL 10-14, 1999 AMERI, XP001002421
- FAIRCLOTH, G. (1) ET AL: "Aplidine (APL) is a novel marine-derived depsipeptide with in vivo antitumor activity." PROCEEDINGS OF THE AMERICAN ASSOCIATION FOR CANCER RESEARCH ANNUAL MEETING, (MARCH, 1998) VOL. 39, PP. 227. MEETING INFO.: 89TH ANNUAL MEETING OF THE AMERICAN ASSOCIATION FOR CANCER RESEARCH NEW ORLEANS, LOUISIANA, USA MARCH 28-APRIL 1, 1998 AMERICAN, XP001002422 cited in the application
- NUIJEN, B. (1) ET AL: "Pharmaceutical development of anticancer agents derived from marine sources." ANTI-CANCER DRUGS, (NOVEMBER, 2000) VOL. 11, NO. 10, PP. 793-811. PRINT., XP001002514
- RAYMOND, ERIC (1) ET AL: "Preliminary results of a phase I and pharmacokinetic study of aplidine given as a 24-hour infusion every 2 weeks in patients with solid tumors and non Hodgkin's lymphomas." PROCEEDINGS OF THE AMERICAN ASSOCIATION FOR CANCER RESEARCH ANNUAL MEETING, (MARCH, 2000) NO. 41, PP. 611. PRINT.. MEETING INFO.: 91ST ANNUAL MEETING OF THE AMERICAN ASSOCIATION FOR CANCER RESEARCH. SAN FRANCISCO, CALIFORNIA, USA APRIL 01-05, 2000, XP001002420
- GELDOF A A ET AL: "Cytotoxicity and neurocytotoxicity of new marine anticancer agents evaluated using in vitro assays." CANCER CHEMOTHERAPY AND PHARMACOLOGY, (1999) 44 (4) 312-8., XP001002505
- DEPENBROCK, H. ET AL: "In vitro activity of aplidine, a new marine-derived anti-cancer compound, on freshly explanted clonogenic human tumour cells and haematopoietic precursor cells." BRITISH JOURNAL OF CANCER, (SEPT., 1998) VOL. 78, NO. 6, PP. 739-744., XP001002515 cited in the application
- FAIRCLOTH, G. (1) ET AL: "Preclinical characterization of aplidine (APD), a new marine anticancer depsipeptide (MADEP." PROCEEDINGS OF THE AMERICAN ASSOCIATION FOR CANCER RESEARCH ANNUAL MEETING, (1997) VOL. 38, NO. 0, PP. 103. MEETING INFO.: EIGHTY-EIGHTH ANNUAL MEETING OF THE AMERICAN ASSOCIATION FOR CANCER RESEARCH SAN DIEGO, CALIFORNIA, USA APRIL 12-16, 1997, XP001002423 cited in the application

## Description

The present invention relates to the treatment of cancers.

### BACKGROUND OF INVENTION

Cancer comprises a group of malignant neoplasms that can be divided into two categories, carcinoma, comprising a majority of the cases observed in the clinics, and other less frequent cancers, which include leukaemia, lymphoma, central nervous system tumours and sarcoma. Carcinomas have their origin in epithelial tissues while sarcomas develop from connective tissues and those structures that had their origin in mesoderm tissues. Sarcomas can affect, for instance, muscle or bone and occur in the bones, bladder, kidneys, liver, lung, parotid or spleen.

Cancer is invasive and tends to metastasise to new sites. It spreads directly into surrounding tissues and also may be disseminated through the lymphatic and circulatory systems. Many treatments are available for cancer, including surgery and radiation for localised disease, and drugs. However, the efficacy of available treatments on many cancer types is limited, and new, improved forms of treatment showing clinical benefit are needed. This is especially true for those patients presenting with advanced and/or metastatic disease. It is also true for patients relapsing with progressive disease after having been previously treated with established therapies for which further treatment with the same therapy is mostly ineffective due to acquisition of resistance or to limitations in administration of the therapies due to associated toxicities.

Chemotherapy plays a significant part in cancer treatment, as it is required for treatment of advanced cancers with distant metastasis and often helpful for tumour reduction before surgery, and many anti-cancer drugs have been developed based on various modes of action.

Dehydrodidemnin B, now known as aplidine, is the subject of WO91/04985.

Further information on aplidine is to be found in, for example:

Jimeno, J., "Exploitation of marine microorganisms and invertebrates: Anticancer drugs from marine origin", IBC Conf Discov Drugs from Nat Novel Approaches New Sources (Dec 8-9, London) 1994,

Faircloth, G. et al., "Dehydrodidemnin B (DDM) a new marine derived anticancer agent (MDA) with activity against experimental tumour models", 9th NCI-EORTC Symp New Drugs Cancer Ther (March 12-15, Amsterdam) 1996, Abst 111

Sakai, R. et al., "Structure-activity relationships of the didemnins", Journal of Medicinal Chemistry 1996, 39 (14): 2819

Urdiales, J.L. et al., "Antiproliferative effect of dehydrodidemnin B (DDB), a depsipeptide isolated from Mediterranean tunicates", Cancer Letters 1996, 102(1-2): 31

Faircloth, G. et al., "Preclinical characterization of aplidine (APD), a new marine anticancer depsipeptide (MADEP)", Proc Amer Assoc Cancer Res 1997, 38: Abst 692

Depenbrock, H. et al., "In vitro activity of aplidine, a new marine-derived anti-cancer compound, on freshly explanted clonogenic human tumour cells and haematopoietic precursor cells", British Journal of Cancer 1998, 78(6) : 739

Faircloth, G. et al., "Aplidine (aplidine) is a novel marine-derived depsipeptide with in vivo antitumour activity", Proc Amer Assoc Cancer Res 1998, 39: Abst 1551

Faircloth, G. et al., "Preclinical development of aplidine, a novel marine-derived agent with potent antitumour activity", 10th NCI-EORTC Symp New Drugs Cancer Ther (June 16-19, Amsterdam) 1998, Abst 129

Mastbergen, S.C. et al., "Cytotoxicity and neurocytoxicity of aplidine, a new marine anticancer agent evaluated using in vitro assays", 10th NCI-EORTC Symp New Drugs Cancer Ther (June 16-19, Amsterdam) 1998, Abst 131

In preclinical studies, aplidine had dose-dependent cytotoxic activity against the two epithelial-like cell lines, CT-1 and CT-2, and the human colon cancer cell line, HT-29. The most proliferative line, CT-2, was the most sensitive to aplidine. In addition the compound decreased ornithine decarboxylase activity in all three cell lines (Lobo C, Garcia-Pozo SG, *et al.* Effect of dehydrodidemnin B on human colon carcinoma cell lines. Anticancer Research. 17: 333-336, Jan-Feb 1997). In a similar study, aplidine 50 nmol/L inhibited the growth of the breast cancer cell lines, MDA-MB231 and MCF-7 by 17 and 47%, respectively.

A significant increase in spermidine and spermine was observed in the treated cells (Gomez-Fabre PM, De Pedro E, et al). Polyamine contents of human breast cancer cells treated with the cytotoxic agents chlorpheniramine and dehydrodidemnin B. Cancer Letters. 113: 141-144, 26 Feb 1997). Flow cytometric analysis showed that aplidine did not induce any apparent cell cycle pertubations (Erba E, Balconi G, et al. Cell cycle phases pertubations induced by new natural marine compounds. Annals of Oncology. 7 (Suppl. 1): 82, 1996). In mice, aplidine was active against implanted P388 leukaemia and B16 melanoma, with an optimal dose of 160 microg/kg. Unlike didemnin B, aplidine was active in SC implanted lewis lung carcinomas (Faircloth G, Rinehart K, *et al.* Dehydrodidemnin B a new marine derived anticancer agent with activity against experimental tumour models. Annals of Oncology. 7 (Suppl. 1): 34, 1996).

Continuous exposure to low concentrations of aplidine inhibited the growth of a number of tumour cell lines, including non-Hodgkin's lymphoma, melanoma and breast, melanoma, ovarian and non-small cell lung cancers. The magnitude of effect was dependent on the time of exposure and appeared to be achievable at non-myelotoxic concentrations. Non-small cell lung cancer, breast cancer and melanoma cell lines were sensitive to a continuous exposure to aplidine at concentrations of >= 0.001 micromol/L. Aplidine had similar toxicity to doxorubicin against clonogenic haematopoietic stem cells (Depenbrock H, Peter R, *et al.* In vitro activity of aplidine, a new marine-derived anti-cancer compound, on freshly explanted clonogenic human tumour cells and haematopoietic precursor cells. British Journal of Cancer. 78: 739-744, No. 6, Sep 1998).

Aplidine had significant activity against mice bearing human cancer xenografts. At a maximum tolerated dose of 2.1 mg/kg, aplidine produced near complete remissions in some animals with a treated/control (T/C) tumour ratio of 9%. At 1.25 mg/kg, significant activity was seen against gastric tumours (T/C 14%) and prostate tumour growth inhibition was also observed (T/C 25%) (Faircloth G, Grant W, *et al.* Preclinical development of aplidine, a novel marine-derived agent with potent antitumour activity. Annals of Oncology. 9 (Suppl. 2): 34, 1998).

### SUMMARY OF INVENTION

We have developed methods to treat human patients with aplidine leading to clinical improvement. The invention relates to a use as claimed in claim 1.

Examples of pharmaceutical compositions include liquid (solutions, suspensions or emulsions) with suitable composition for intravenous administration, and they may contain the pure compound or in combination with any carrier or other pharmacologically active compounds.

The compound aplidine may be used with other drugs to provide a combination therapy. The other drugs may form part of the same composition, or be provided as a separate composition for administration at the same time or a different time. The identity of the other drug is not particularly limited, and suitable candidates include:
a) drugs with antimitotic effects, especially those which target cytoskeletal elements, including microtubule modulators such as taxane drugs (such as taxol, paclitaxel, taxotere, docetaxel), podophylotoxins or vinca alkaloids (vincristine, vinblastine);
b) antimetabolite drugs (such as 5-fluorouracil, cytarabine, gemcitabine, purine analogues such as pentostatin, methotrexate);
c) alkylating agents or nitrogen mustards (such as nitrosoureas, cyclophosphamide or ifosphamide);
d) drugs which target DNA such as the antracycline drugs adriamycin, doxorubicin, pharmorubicin or epirubicin;
e) drugs with target topoisomerases such as etoposide;
f) hormones and hormone agonists or antagonists such as estrogens, antiestrogens (tamoxifen and related compounds) and androgens, flutamide, leuprorelin, goserelin, cyprotrone or octreotide;
g) drugs which target signal transduction in tumour cells including antibody derivatives such as herceptin;
h) alkylating drugs such as platinum drugs (cis-platin, carbonplatin, oxaliplatin, paraplidineatin) or nitrosoureas;
i) drugs potentially affecting metastasis of tumours such as matrix metalloproteinase inhibitors;
j) gene therapy and antisense agents;
k) antibody therapeutics;
l) other bioactive compounds of marine origin, notably kahalalide F or the ecteinascidins such as et-743;
m) skeletal muscle protectors such as carnitine supplements;
o) other drugs which combat side effects of aplidine such as antiemetics;
p) more generally drugs which allow aplidine to be dosed at the Recommended Dose and manage toxicity.

We have further found that aplidine inhibits expression of the gene (FLT1) encoding the receptor of the Vascular Endothelial Growth Factor (VEGF).

In addition, aplidine has been found to severely inhibit production of the VEGF protein itself by tumour cells.

VEGF secretion by a cell mass, in particular a tumour cell mass, causes de novo vascularization (angiogenesis) leading to new blood vessels forming towards the cell mass and establishing a network of capillaries that is able to supply it with irrigation for its sustained proliferation. These effects, in particular the demonstrated abolition of production of VEGF by tumour cells are expected to severely inhibit the ability of the tumour cells to bring forth angiogenesis. In addition, VEGF is required directly by some hematopoietic tumour cells (such as MOLT4 human leukaemia cells) as a growth factor.

Thus aplidine can be predicted to have an inhibitory effect on de novo vascularization of growing primary tumours or metastases, therefore inhibiting growth of the tumours, which are known to require vascularization for growth. Aplidine should also be active on hematopoietic tumours.

In addition Aplidine is known to modulate Calcium channel function in cells.

Bladder tumours are one type of tumour over-expressing the receptor to Epithelial Growth Factor (EGF), which leads to upregulation of VEGF and the VEGF receptor. Binding of VEGF to its receptor is believed to lead to cell growth stimulation by means of transitory local calcium ion changes among other mechanisms for signalling. A compound inhibiting VEGF action is expected to be inhibitory to such tumours.

Experimentally, aplidine has been found to have exceedingly high activity on human bladder cancer (giving complete remissions in some animal models), in accordance with the prediction.

Aplidine can be predicted to have a broad spectrum antitumour activity due to its effects on a large number of tumours.

The effect of VEGF is more relevant because it involves an inhibition of new blood vessels. In addition to effects on blood vessels, certain tumours required VEGF directly for cell growth (i.e. leukaemia, lymphomas, bladder tumours and ovarian tumours).

According, we envisage a method of treatment of a tumour that is dependent on the angiogenic process which involves administration of aplidine. The invention further provides a process for preparing an inhibitor of cancer invasion or cancer metastasis comprising admixing an effective dose of aplidine with a pharmaceutically acceptable carrier.

In particular, we envisage methods of treating angiogenesis disorders such as neoplasia including metastasis.

Responses in cancer patients have been observed in clinical trials with aplidine, demonstrating usefulness of the method of treatment.

Phase I clinical studies and pharmacokinetic analysis demonstrate that aplidine presents a positive therapeutic window with manageable toxicity in the range of dosage required for clinical efficacy in the treatment of cancer patients.

Aplidine is supplied and stored as a sterile lyophilised product, consisting of aplidine and excipient in a formulation adequate for therapeutic use.

Solubilised aplidine shows substantial degradation under heat and light stress testing conditions, and a lyophilised dosage form was developed, see WO99/42125. In a currently preferred embodiment freeze-drying was performed from a 500 mg/mL solution of aplidine in 40% (v/v) *tert*-butanol in Water for Injection (WfI) containing 25 mg/mL D-mannitol as bulking agent. The prototype, containing 500 mg aplidine and 25 mg D-mannitol as bulking agent per vial was found to be the optimal formulation in terms of solubility, length of lyophilisation cycle and dosage requirements in the clinical studies. The optimal reconstitution solution was found to be 15/15/70% (v/v/v) Cremaphor EL/ethanol/WfI (CEW). Both reconstituted product and dilutions (up to 1:100 v/v) of the reconstituted product with normal saline appeared to be stable for at least 24 hours after preparation. Shelf life data, available thus far, show that the formulation is stable for at least 1 year when stored at 4°C in the dark.

Preparation of the infusion solution is also performed under aseptic conditions by withdrawing the reconstituted solution volume corresponding to dosage calculated for each patient, and slowly injecting the required reconstituted solution volume into an infusion bag or bottle containing between 100 and 1000 ml of 0.9% sodium chloride, after which the whole is homogenised by slow manual shaking. The aplidine infusion solution should be administered intravenously, as soon as possible, within 48 hours after preparation. PVC and polyethylene infusion systems, as well as clear glass are preferred container and conduit materials.

The administration is performed in cycles. Dose delays and/or dose reductions and schedule adjustments are performed as needed depending on individual patient tolerance of treatments, in particular dose reductions are recommended for patients with higher than normal serum levels of liver transaminases or alkaline phosphatase, or bilrubin.

The Recommended Dose (RD) is the highest dose which can be safely administered to a patient producing tolerable, manageable and reversibly toxicity according to the Common Toxicity Criteria established by the National Cancer Institute, (USA) with no more than 2 out of 6 patients presenting any dose limiting toxicities (DLT).

Guidelines for cancer therapy frequently call for administration of chemotherapeutic agents at the highest safe dose at which toxicity is manageable in order to achieve maximum efficacy (DeVita, V.T. Jr., Hellman, S. and Rosenberg, S.A., Cancer: Principles and Practice of Oncology, 3rd ed., 1989, Lipincott, Philadelphia).

DLTs for aplidine were determined in clinical studies. These studies established a recommended dose level for differing kinds of dosing protocols.

Aplidine can be safely administered at a dosage level at or below the Recommended Dose (RD).

Infusion regimes of this invention are the following:
24 hour infusion weekly for a number of weeks, say three weeks, followed by one week rest;
biweekly 24 hour infusion;
1 hour infusion weekly for 3 weeks every 4 weeks.

In particular, intravenous infusion can be carried out as a 24 hours infusion once a week for 3 weeks q 4 weeks. More data is given in Examples 3, 4, 10 and 11. A Recommended Dose of 3750 µg/m²/wk x 3 seems to be appropriate. This protocol has been amended and patients will now be treated using a different schedule which seems feasible: 3 hour infusion every 2 weeks with no rest. See Example 11. In the 24 h biweekly study patients are being treated at 7000 µg/m²/2wks. See examples 6, 13 and 16. Patients included in the study lh/wk x 3 every 4 weeks are being treated at doses up to 3600 µg/m² /wk x 3 wks. See examples 12 and 15. When aplidine is used in combination with other therapeutic agents, the dosages of both agents may need to be adjusted.

Previously the principal biological responses reported to the administration of aplidine had been observed in animal or *in vitro* models, known to be notoriously inaccurate concerning their usefulness to predict responses in human patients, or in human patients in experimental settings where an effective, safe method of treatment was unavailable (either the dosage used was a toxic dose significantly elevated over the recommended dose or the administration schedule was not appropriate).

In clinical trials using the method of this invention, appropriate plasma levels were achieved in patients at RD, and most importantly, objectively measurable responses demonstrated evidence of clinical benefit to patients.

Definitions for patient toxicities are adopted from WHO Criteria and the responses determined following WHO Response Criteria.

Objective responses were obtained in patients with advanced and/or metastatic cancers refractory to previous treatments, which included those described in the Examples.

In particular treatment with this method has shown responses in cancer patients with advanced and/or metastatic disease, which exhibited progressive disease after having been previously treated with established therapies.

A preferred method therefore involves identifying cancer patients who have been treated for cancer, particularly patients who have received chemotherapy, and treating them with aplidine.

### DESCRIPTION OF THE DRAWINGS

Figure 1 shows the decrease in flt-1 expression observed using microarrays confirmed by RT-PCR analysis.
Figure 2 shows the decrease in flt-1 mRNA induced by aplidine in MOLT-4 cells.
Figures 3a and 3b show the VEGF-Flt-1 Autocrine Loop in MOLT-4 Cells and the effect of aplidine.
Figure 4 shows aplidine Blocks VEGF Secretion from MOLT-4 Cells.
Figure 5 shows aplidine induced block of VEGF secretion.
Figure 6 shows a strong decrease in the levels of VEGF mRNA in MOLT-4 cells.
Figure 7 shows aplidine dose not decrease the activity of VEGF promoter transfected in MOLT-4 cells
Figure 8 shows aplidine does not block the binding of HIF-1 and AP-1 transcription factors to their consensus DNA sequences present in the VEGF promoter
Figure 9 shows aplidine does not block the binding of HIF-1 transcription factor to their consensus DNA sequences present in the VEGF promoter.
Figures 10a and 10b show VEGF added in the culture medium of MOLT-4 cells slightly reduced the activity of low concentrations of aplidine while at high concentrations is without effects.
Figure 11 shows aplidine is able to reduce the secretion of VEGF from the human ovarian cancer line IGROV-1.
Figure 12 shows aplidine reduces the mRNA levels of VEGF also in the human ovarian cancer line IGROV-1.
Figure 13 shows aplidine does not affect the promoter activity of VEGF measured using the luciferase/renilla reporter gene system.
Figure 14 shows a dose AUC relationship.
Figure 15a and 15b show activity in Medullary Thyroid Cancer: ACE Levels

### EXAMPLES

### Example 1 (Comparative example)

Gene expression profile in human leukenic MOLT4 cells treated with the marine compound aplidine

The early changes in gene expression induced by aplidine in MOLT-4 cells were evaluated by using cDNA expression arrays (Atlas Human Cancer, Clontech). MOLT-4 cells were treated for 1 hour with concentrations of aplidine which inhibit the growth by 50% and total RNA was isolated at 0, 1, 6 and 24 hours after drug wash out. Filters were hybridised with equal amount of 32P labelled cDNA. Analysis of the results was carried out using ATLAS IMAGE 1.0 software. Changes in gene expression greater than 2 fold were taken as significant changes in RNA expression and subsequently confirmed by PCR. A marked time-dependent reduction in the expression of VEGF-R1 (flt-1) was observed and confirmed at RNA level by PCR and at protein level by Western blotting.

### Example 2 (Comparative example)

Correlation of selective antitumour activites of the marine-derived compound aplidine using different model systems

Different model systems were evaluated to provide the basis for further clinical work. Selective antitumour activities were seen against two histologically different solid tumours: human gastric and prostate carcinomas. Potent *in vitro* activity to primary gastric tumour specimens or Hs746T gastric tumour cells is evident with IC₅₀ values of 146 and 450 pM, respectively. A less potent, but no less selective, IC₅₀ activity of 3.4 nM was determined against PC-3 prostate tumour cells. *In vitro* activities were evaluated in nude rodents using sc implanted tumour fragments or hollow fibres (HF) containing tumour cells.

**Table 1. Optimal Dose and In Vivo Activities of aplidine**

| Tumour | Line | Regimen | sc Model | Animal | Dose (mg/kg) | Activity (%T/C) |
|---|---|---|---|---|---|---|
| Gastric | MRI- | qd9.ip | xenograft | mouse | 2.1 | 19% |
| | H254 | | | | 1.05 | 17% |
| | | q4dx3.ip | xenograft | mouse | 1.25 | 18% |
| | | 24 hr.iv | HF | rat | 0.7 | 20% |
| | | inf. | | | | |
| Prostate | PC-3 | qd9.ip | xenograft | mouse | 1.25 | 25% |
| | | | | | 0.62 | 30% |
| | | q4dx3.ip | xenograft | mouse | 2.10 | 34% |
| | | | | | 1.05 | 38% |
| | | 24 hr.iv | HF | rat | 0.70 | 31% |
| | | inf. | HF | rat | 0.70 | 33% |
| | | 5 day, iv | | | | |
| | | inf | | | | |

Optimal activities were observed in xenografted gastric (17-20%) and prostate (25-38%) tumours following *ip* administration. Follow-up studies necessitated using rats for *iv* infusions. With this variation, a 24 hr *iv* infusion schedule produced similar activities against HF gastric (20%) and HF prostate (31%) tumour cells. Cytotoxicity was also found using a 5 day *iv* infusion schedule against HF prostate tumour cells (33%). The extended *in vivo* evaluations not only show that there is a strong relative correlation to the *in vitro* cytotoxic profile, but also a strong correlation with *in vivo* models used to characterise the tumour selectivity that identified aplidine as a candidate for clinical development.

### Example 3

A phase I and pharmacokinetic study of aplidine given as a weekly 24 hours infusion in patients with advanced solid tumours

*In vivo* studies revealed that *in vivo* activity increased by prolonging infusion duration. In this study 16 patients were treated. patients characteristics: median age 55 years, median PS 1, male/female 11/5, tumour types being as follows: Head and neck 5, kidney 2, colon 3, rectum 2, sarcoma 1 and melanoma 3, all pre-treated with chemotherapy (median 2 lines).

Aplidine was administered as a 24 h infusion at the following dose levels (Dls): 133 (3 pts), 266 (3 pts), 532 (3 pts), 1000 (3 pts), 2000 (3 pts) and 3000 (1 pt) mcg/m²/wk x 3 every 28 days.

No dose limiting toxicities (DLTs) were observed. Only mild non-haematological toxicities consisting of nausea g 1, mucositis g 1, asthenia g 1 were reported. Phlebitis of the infusion arm was common and concentration-dependent. Pharmacokinetic analysis was performed in all patients, showing plasma levels at the DLs 1000 mcg/m²/w and 2000 mcg/m²/w equivalent to the active in *vitro* concentration (1 ng/ml). At DL 532 mcg/m²/w clinical improvement was observed in 1 pre-treated patient with advanced melanoma.

### Example 4

Phase I and pharmacokinetic (PK) study of aplidine (APL) using a 24 hour, weekly schedule.

To date, 25 pts (median age 58 yrs, median ECOG 1) with advanced, previously treated solid tumours or lymphoma have been treated in this phase I study. Using a schedule of APL 24 h weekly x 3 followed by 1 wk rest, the following dose levels have been tested: 133 (n-3 pts), 266 (3), 532 (3), 1000 (3), 2000 (3), 4500 (3) and 3750 µg/m²/wk (3). With 60 cycles (180 infusions) administered, all pts are evaluable for toxicity. The Maximum Tolerated Dose was 4500 µg/m²/wk x 3 with grade (G) 3 muscular toxicity (biopsy proven type II muscular atrophy). G4 CPK and G3 transaminitis the dose-limiting toxicities in 2 or 4 pts. G2 malaise was observed in most pts and G2/3 emesis at ≥ 2000 µg/m² phlebitis is common but concentration-dependent. All pts have been sampled for PK analysis by LC/MS/MS. Preliminary data indicate extensive tissue distribution, a long elimination t ½ of 10-24 h nad plasma levels > 1 ng/ml (which is active *in vitro*)*.* One pt with advanced melanoma resistant to DTIC/interferon had a clinical improvement maintained for > 30 weeks. This weekly infusional study demonstrates the feasibility and activity of a dose-dense APL schedule. As expected, neuromuscular toxicity is dose-limiting. The possible recommended dose of 3750 µg/m²/wk x 3 is being assessed.

### Example 5

Clinical pharmacokinetics (PK) of aplidine (APL) in patients with solid tumours and non-Hodgkin lymphomas.

Four intravenous schedules are under phase I evaluation: weekly 24 h infusion, biweekly 24 h infusion and 5 consecutive days 1 h infusion every 3 weeks. APL blood concentrations are analysed by liquid chromatography-tandem mass spectrometry. Initial results show accumulation in blood cells and a plasma PK characterised by na extensive distribution (volume of distribution usually in excess of 200 L/m²) and elimination half lives in the order of 10 to 24 h. An open 2-compartment model fits appropriately most profiles after 24 h infusion. For 1 h infusions a 3-compartment model provides a better fit in most cases. Obtained plasma levels are known to be active in *vitro.* Evaluation of additional patients, and a comparison of blood cells and plasma PK is ongoing.

### Example 6

Preliminary results of a phase I and pharmacokinetic study of aplidine given as a 24 hour infusion every 2 weeks in patients with solid tumours and non-Hodgkins lymphomas.

Aplidine is given as a 24 hour infusion every 2 weeks. The starting dose was 200 µg/m²/d and dose escalation proceed including so far 400, 800, 1600, and 3200 µg/m²/d. A total of 18 patients (M/F: 7/11, median age 52, OMS 0/1: 10/8) have been entered. So far, no dose limiting toxicity was observed. Among evaluable patients, toxicity consisted of mild grade I-II nausea/vomiting, grade I-II asthenia, and cramping occurring during or immediately after the infusion. No neuromuscular toxicity were reported at the evaluated doses. One patient with an advanced lung cancer and documented tumour progression at the dose of 1600 µg/m² developed a haemolytic anaemia and thrombocytopenia that was considered unlikely related to the study drug. Initial pharmacokinetic analysis showed that the drug is extensively distributed and blood concentrations. An open 2-comarptment model fits appropriately most plasma concentration profiles. The terminal half-life is usually in the order of 10-24 h. Clinical improvement was seen in a patient with a non-Hodgkin lymphoma. The accrual is ongoing to determine the dose limiting toxicity and the dose to be recommended in phase II studies.

### Example 7 (Comparative example)

Gene expression profile in human lekemic MOLT4 cells treated with the marine compound aplidine.

In the present study we have evaluated the early changes in gene expression induced by aplidine in MOLT-4 cells by using cDNA expression arrays (Atlas Human Cancer, Clontech). MOLT-4 cells were treated for 1 hour with concentrations of aplidine which inhibit the growth by 50% and total RNA was isolated t 0, 1, 6 and 24 hours after drug wash out. Filters were hybridised with equal amounts of 32P labelled cDNA. Analysis of the results was carried out using ATLAS IMAGE 1.0 software. Changes in gene expression greater than 2 fold were taken as significant changes in RNA expression and subsequently confirmed by PCR. A marked time-dependent reduction in the expression of VEGF-R1 (flt-1) was observed and confirmed at the RNA level by PCR and at the protein level by Western blotting. Whether the down-regulation of flt-1 is involved in the cytotoxic and antitumour effect of aplidine is under investigation at the moment. Also the characterisation of the expression of other genes which appear to be down-regulated after aplidine exposure is currently underway.

Quantitative Changes in Gene Expression Induced by Aplidine in MOLT-4 Cells

| Gene | 1 Hour | 6 Hours | 24 Hours |
|---|---|---|---|
| | | | |
| B-RAF | - | +2.0 | +2.6 |
| FLT-1 | -1.5 | 5.0 | -3.4 |
| FMS | - | +2.7 | +2.1 |
| ETR | +2.7 | - | - |
| DNA-PK | - | +3.0 | +3.8 |
| PLK-1 | - | +3.0 | +4.4 |

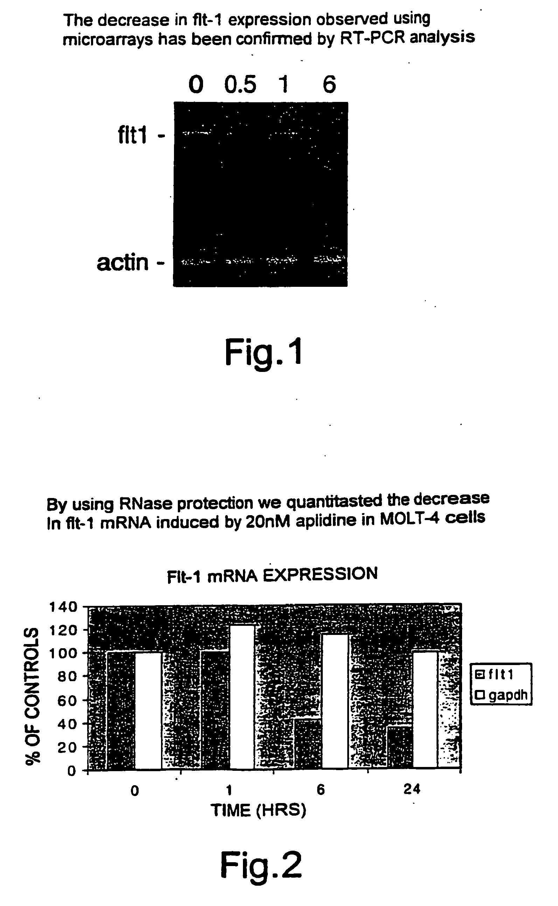

The decrease in flt-1 expression observed using microarrays was confirmed by RT-PCR analysis, see Figure 1.

By using RNase protection we quantitated the decrease in flt-1 mRNA induced by 20 nM aplidine in MOLT-4 cells, see Figure 2.

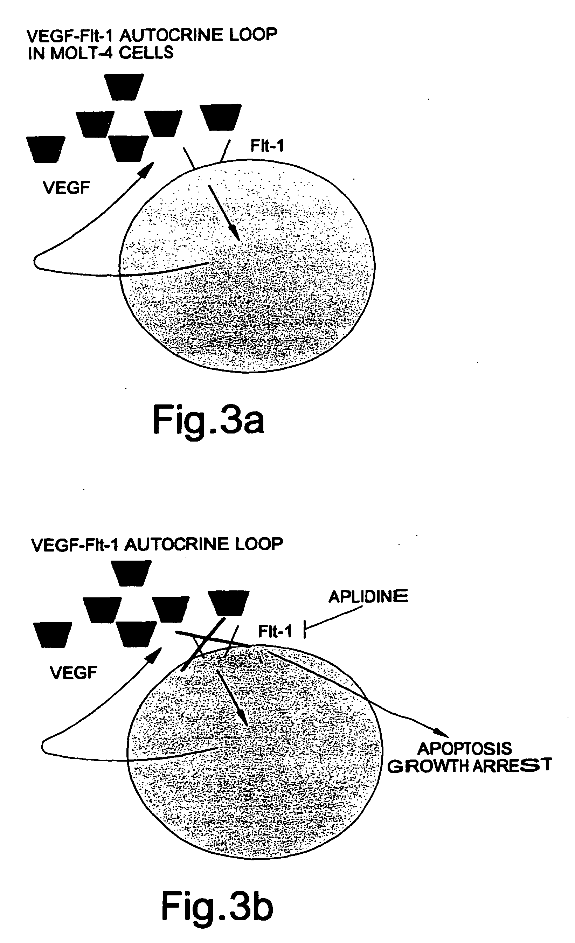

Figures 3a and 3b show the VEGF-Flt-1 Autocrine Loop in MOLT-4 Cens and the effect of aplidine in the VEGF-Flt-1 Autocrine Loop.

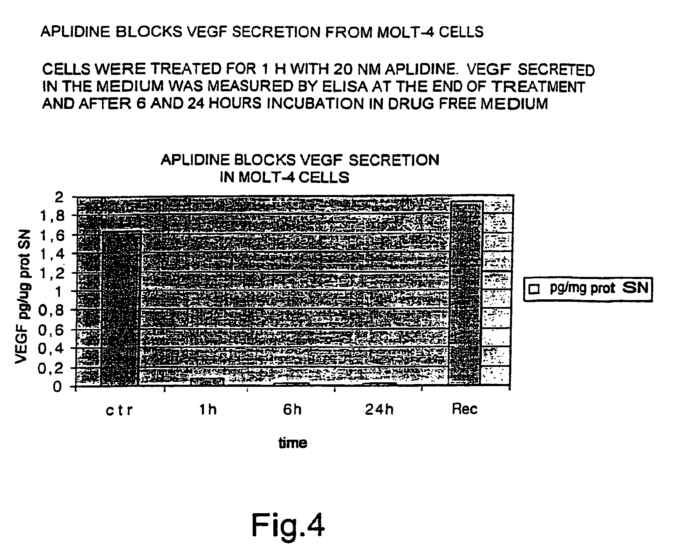

Aplidine Blocks VEGF Secretion from MOLT-4 Cells, see Figure 4. Cells were treated for 1 h with 20 nM aplidine. VEGF secreted in the medium was measured by ELISA at the end of treatment and after 6 and 24 hours incubation in drug-free medium.

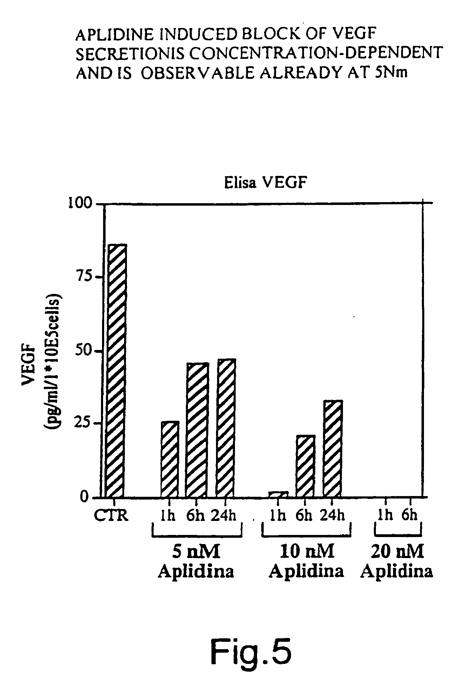

Aplidine induced block of VEGF secretion is concentration-dependent and is observable already at 5nm, see Figure 5.

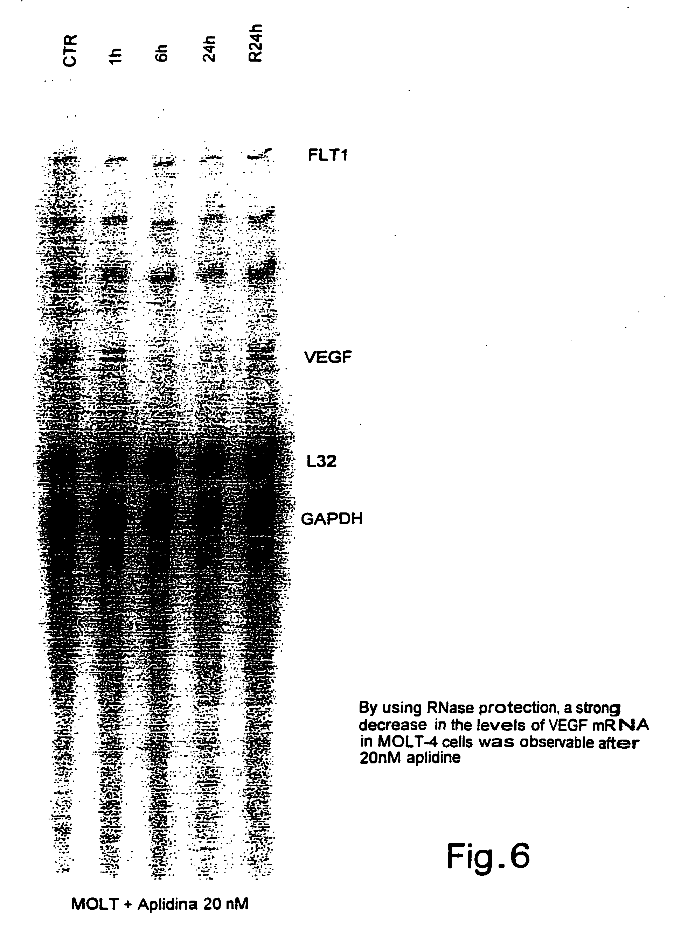

By using Rnase protection, a strong decrease in the levels of VEGF mRNA in MOLT-4 cells was observable after 20nM aplidine, see Figure 6.

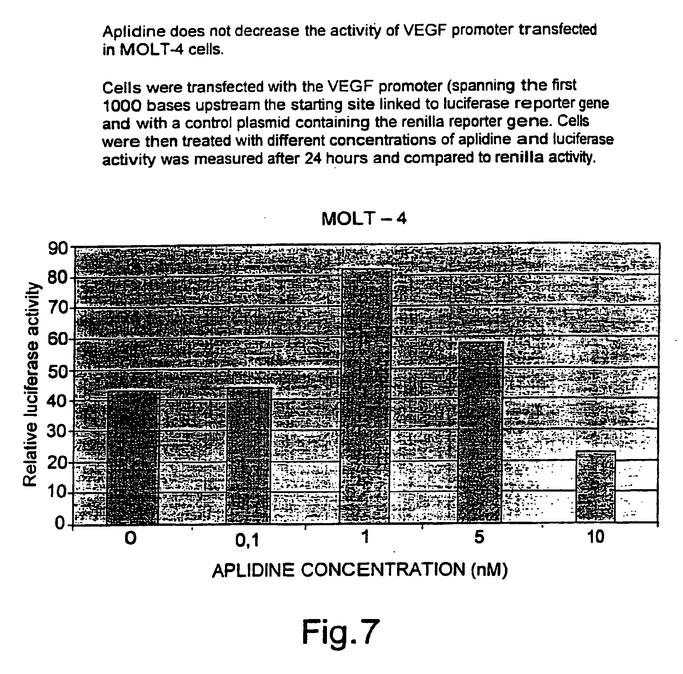

Aplidine dose not decrease the activity of VEGF promoter transfected in MOLT-4 cells, see Figure 7. Cells were transfected with the VEGF promoter (spanning the first 1000 bases upstream the starting site linked to luciferase reporter gene and with a control plasmid containing the renilla reporter gene. Cells were then treated with different concentrations of aplidine and luciferase activity was measured after 24 hours and compared to renilla activity.

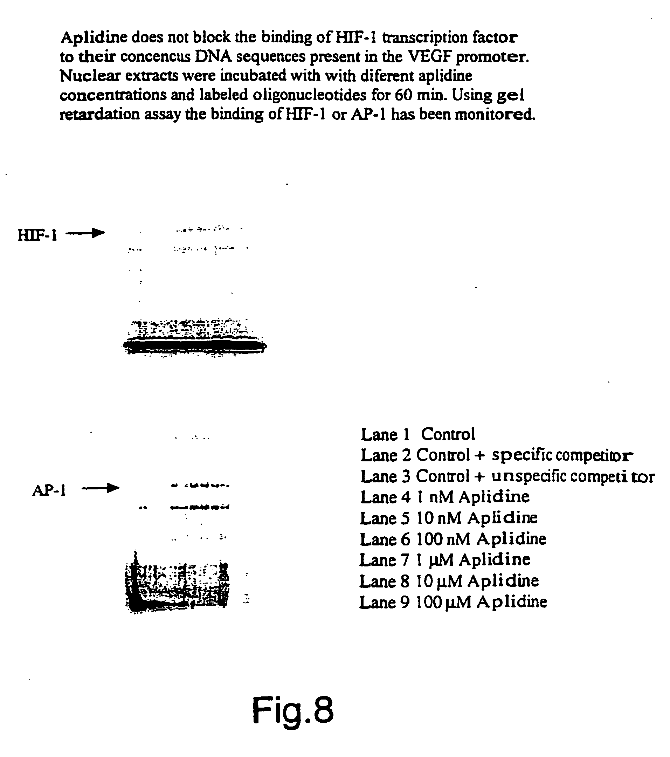

Aplidine does not block the binding of HIF-1 and AP-1 transcription factors to their consensus DNA sequences present in the VEGF promoter, see figure 8. Nuclear extracts were incubated with different aplidine concentrations and labelled oligonucleotides for 60 min. Using gel retardation assay the binding of HIF-1 or AP-1 has been monitored.

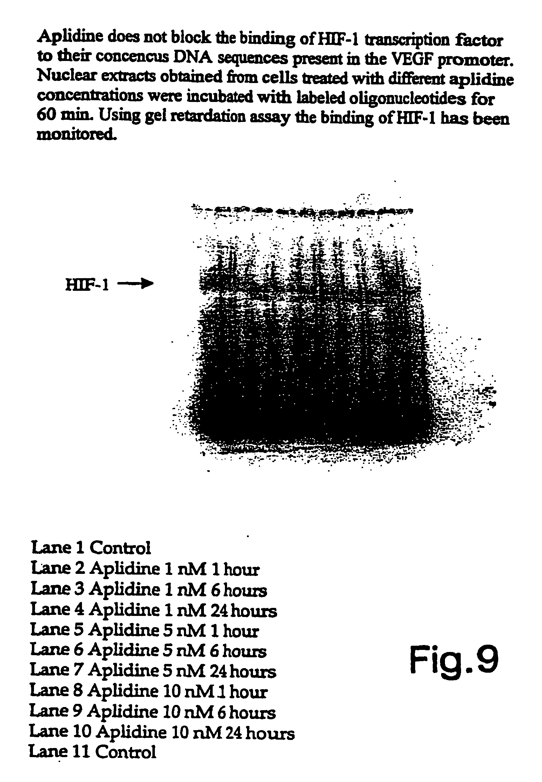

Aplidine does not block the binding of HIF-1 transcription factor to their consensus DNA sequences present in the VEGF promoter, see figure 9. Nuclear extracts obtained from cells treated with different aplidine concentrations were incubated with labelled oligonucleotides for 60 min. Using gel retardation assay the binding of HIF-1 has been monitored.

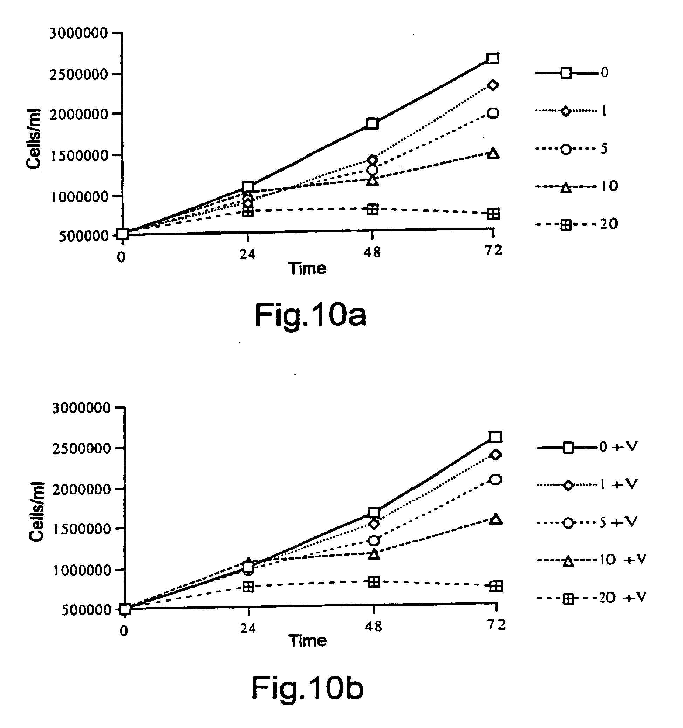

VEGF (10 ng/ml) added in the culture medium of MOLT-4 cells cultured in 10% FCS slightly reduced the activity of low concentrations of aplidine while at high concentrations is without effects, see Figures 10a, 10b.

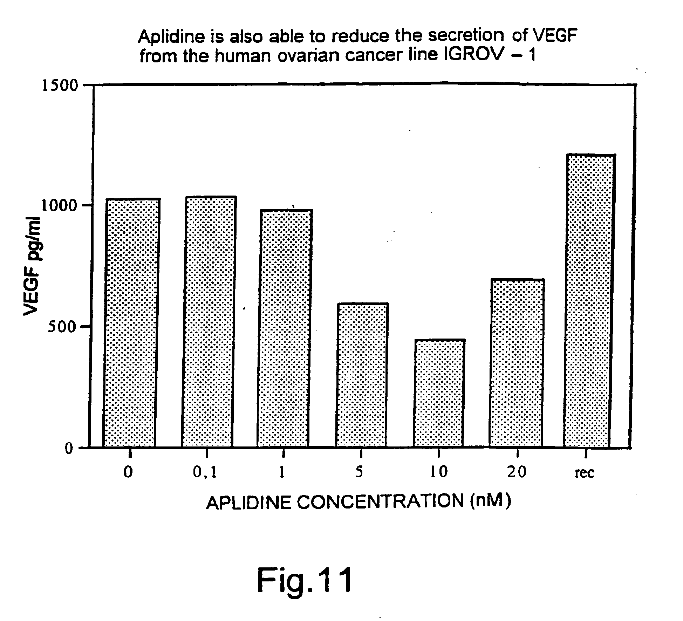

Aplidine is also able to reduce the secretion of VEGF from the human ovarian cancer line IGROV-1, see figure 11.

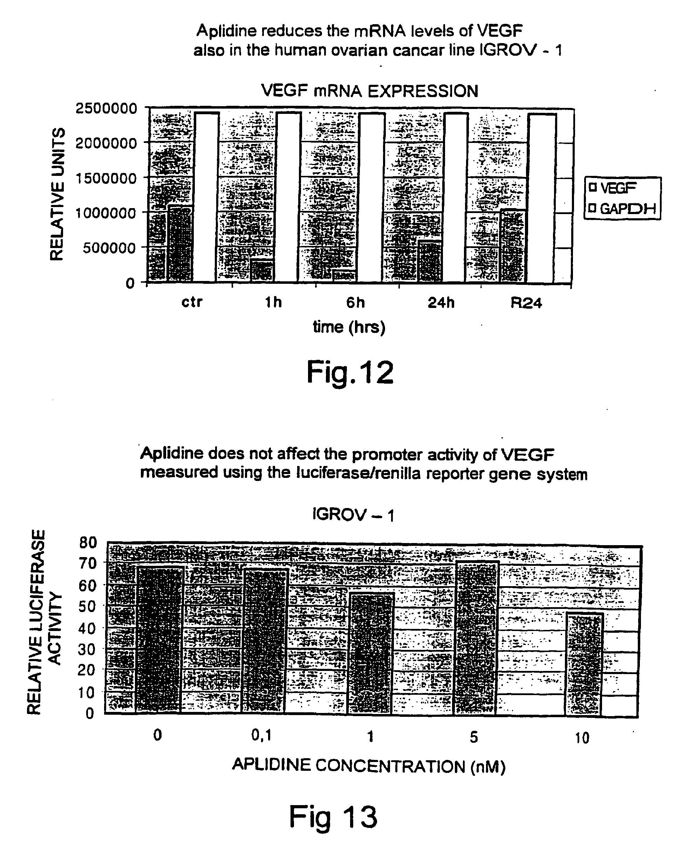

Aplidine reduces the mRNA levels of VEGF also in the human ovarian cancer line IGROV-1, see figure 12.

Aplidine does not affect the promoter activity of VEGF measured using the luciferase/renilla reporter gene system, see figure 13.

### Example 8 (Comparative example)

Aplidine blocks VEGF secretion and VEGF/VEGF-R1 autocrine loop in a human leukemic cell line

Aplidine was found to induce strong apoptosis in the human leukaemia cell line MOLT-4. In the same cell line microarray analysis revealed changes in the expression of different genes at early times after treatment. Among these we found that the VEGF-R1 (fit-1) was downregulated by drug treatment and its downregulation was confirmed by northern and western blotting analysis. Further studies showed that treatment of the same cellular system with the compound resulted in a strong reduction of VEGF secretion in the medium. The decrease in VEGF secretion was associated to a decrease in the mRNA encoding for VEGF in MOLT-4 cells treated with aplidine. Trying to elucidate the mechanism by which aplidine blocks VEGF secretion, we found that the compound does not change the half life of VEGF mRNA. Similarly, using the electromobility shift assay, aplidine did not change the ability of two transcription factors, HIF-1 and AP-1 to bind their respective consensus sequence present in the promoter of VEGF and did not decrease the transcription of VEGF when a VEGF promoter-luciferase construct was used in transient transfection experiments. The decreased secretion of aplidine was associated with an increased intracellular accumulation of VEGF strongly suggesting that the compound could act through a block of the secretion of VEGF. Simultaneous treatment of MOLT-4 cells with low concentrations of aplidine and VEGF partially abolish the activity of aplidine suggesting that the drug could partially exert its activity in this cellular system by blocking the autocrine loop VEGF/VEGF-RI.

### Example 9 (Comparative example)

*In vitro* safety profile of aplidine, a marine natural product with chemotherapeutic potential

Using the CellTiter96 (MTS, Promega) *in vitro* cytotoxicity assay, aplidine exhibits little liver (AML-12) or cardiac toxicity (H9 c2 (2-1); LD₅₀ of 1 µM). In contrast, aplidine is very toxic to skeletal muscle (L8), and kidney (NRK-52E) cells (LD₅₀ of 0.1 nM), with intermediate toxicity to myelogenous stem cells (FDC-P1, LDso of 0.1 µM) in close agreement with clinical toxicity data. In fact, the dose limiting toxicity in humans is skeletal muscle atrophy.

Aplidine exhibits neurotoxicity at higher *in vitro* concentrations. Using a fluorescent viability stain (ethidium homodimer and calcein AM, Molecular Probes) coupled with immunocytochemistry, we observed that approximately 1 µM aplidine is toxic to brain cells (both to neurons and astrocytes) and motor (choline acetyl transferatse positive) neurons in the spinal cord, but not substance P positive sensory neurones. The motor neuron sensitivity may help to explain the type II muscle atrophy observed (as predicted) in a small group of patients where the AUC concentration of the drug is elevated due to diminished excretion.

### Example 10

Phase I clinical and pharmacokinetic study of aplidine a new marine didemnin, administered as a 24 hour infusion weekly

A phase I trial was performed using 24 hour infusion weekly x 3 followed by 1 wk rest. 32 patients (median age 58 yrs, median ECOG 1) with advanced, previously treated solid tumours have been treated. They have received 64 courses (median/pt: 2 (1-6)) across 8 dose levels: 133 (3 pts), 266 (3 pts), 532 (3 pts), 1000 (3 pts), 2000 (3 pts), 3000 (3 pts), 4500 (4 pts) and 3750 mcg/m²/wk (10 pts). 2 out of 3 evaluable patients had DLT at 4500 mcg/m²/wk: grade (G) 4 reversible neuromuscular toxicity (biopsy prevent type II muscular atrophy) and G4 CK increase (1 pt), and transient G3 transaminitis (1 pt). Other toxicities included G 1-2 malaise (most patients treated at ≥ 3000 mcg/m²/wk), muscle cramps, G 1-2 emesis (responsive to antiemetics) and injection site reaction (very common and concentration-dependent). All patients have been sampled for PK analysis by a LC/MS/ MS method. Pharmacokinetics are linear and the profiles fit a 2 compartment open model. The drug has extensive tissue distribution (Vss=611 L), high clearance (0.47 L/min) and an elimination t 1/2 of 18.8 h. Maintained plasma levels > 1 ng/ml (active *in vitro*) were obtained at doses ≥3000 mcg/m². One patient with advanced melanoma resistant to DTIC/interferon had definite clinical improvement maintained for > 30 weeks. Four additional patients had minor responses or stable disease for ≥ 4 months . In conclusion, the DLTs of aplidine administered on a weekly infusion schedule were reversible muscle toxicity and transaminitis, which were observed MTD of 4500 mcg/M²/wk. The recommended dose for future trials, 3750 mcg/m²/wk x 3, administered through a central vein catheter, is feasible and associated with mild toxicity.

### Example 11

Phase I clinical and pharmacokinetic study of aplidine a new marine didemnin, administered as a 24 hour infusion weekly

### Patient Characteristics

| | | | |
|---|---|---|---|
| Number of patients | 35 | Tumour type | |
| Gender (male/female) | 23/12 | Colorectal | 12 |
| Median age, years | 56.5 (29-74) | Kidney | 6 |
| (range) | | | |
| ECOG performance | | Head and neck | 5 |
| status | | | |
| 0 | 12 | Melanoma | 4 |
| 1 | 18 | Gastric | 2 |
| 2 | 5 | Breast | 1 |
| Previous radiotherapy | | Lung | 1 |
| Previous | | Soft tissue | 1 |
| chemotherapy | | sarcoma | |
| None | 4 | Lymphoma | 1 |
| 1 regimen | 9 | Thyroid | 1 |
| 2 regimens | 12 | C. unknown origin | 1 |
| 3 ≥ regimens | 10 | | |
| Sites of disease | | | |
| 1 | 14 | | |
| 2 | 12 | | |
| ≥3 | 9 | | |

### Patient Accrual

| Dose level | Dose (mcg/m²/wk) | No. patients | No. Cycles (range) |
|---|---|---|---|
| I | 133 | 3 | 9 (1-6) |
| II | 266 | 3 | 9 (2.5) |
| III | 532 | 3 | 10 (2-6) |
| IV | 1000 | 3 | 7 (1-4) |
| V | 2000 | 3 | 6 (2-2) |
| VI | 3000 | 3 | 4 (1-2) |
| VII | 4500 | 4 | 5 (1-2) |
| VIII | 3750 | 13 | 21+ (1-4) |
| Total | | 35 | 71+ |

### Worst Toxicities Per Patient

| Dose level | | I | II | III | IV | V | VI | VII (MTD) | VIII (RD) |
|---|---|---|---|---|---|---|---|---|---|
| No. patients | | 3 | 3 | 3 | 3 | 3 | 3 | 4 | 13 |
| Nausea | G2 | | | | | 1 | | | 2 |
| | G3 | | | | | | 1 | | |
| Asthenia | G2 | | | 1 | | | 1 | 1 | 3 |
| | G3 | | | | | | 1 | | |
| Inj. Site reaction | | | 1 | | 1 | 1 | 1 | 1 | 3 |
| | G2 | | | | | | | | |
| Myositis | G3 | | | | | | | 1 | |
| CPK elevation | G4 | | | | | | | 1 | |
| Transaminitis | G3 | | | | | | | 1 | 2 |
| | G4 | | | | | | | | 1 |
| Hypersensitivity | G3 | | | | | | | | 1 |

### Antitumour Active

Patient #008 - (Madrid) Highly pre-treated non measurable metastatic melanoma clinical improvement and tumour shrinkage was observed. A biopsy of one of the metastatic lesions revealed no evidence of residual tumour tissue.

Patient #032 - (Madrid) Renal carcinoma: 20% tumour shrinkage

Patient #034 - (Madrid) Thyroid medullary carcinoma. Clinical improvement and SD at lung lymphangitis. Decrease in ACE marker.

### Pharmacokinetics

Plasma aplidine concentrations were determined by liquid chromatography/tandem mass spectography with a limit of quantitation of 0.25 ng/mL and a broad linear range up to 16.00 ng/mL
A total of 15 samples were drawn up to 24 hours after the end of infusion
Dose-linear pharmacokinetics
High Cl median ((quartiles) 0.47(0.40-0.56) L/min) and interpatient variability (coefficient of variation of clearance (Cl), 45%)
Intermediate to long half-life (t ½) (median (quartiles) 18.8 (15-3-25.4) h) Extensive distribution with supraphysiological volume of distribution (Vss) (median (quartiles) 611 (434-733) L)
Blood cell accumulation (2-8 fold as compared to plasma)
Profiles fit a 2-compartment open model

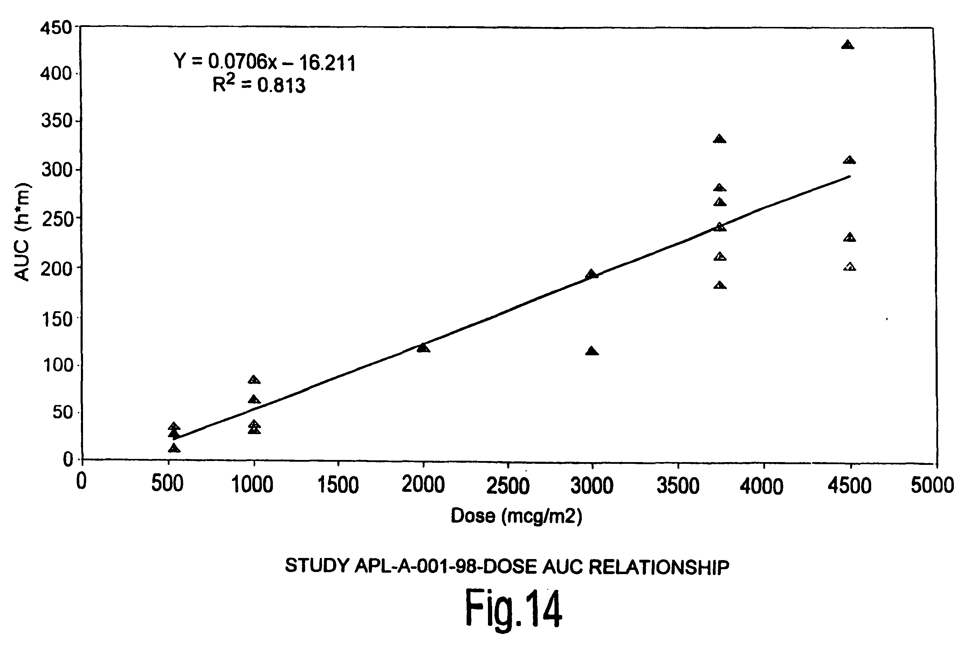

Figure 14 shows the dose AUC relationship.

### Conclusions

DLTs of aplidine administered using this schedule were reversible muscle toxicity and transaminitis observed at the MTD of 4500 mcg/m²/week x 3
The Recommended Dose for future trials, 3750 mcg/m²/week x 3 is feasible and associated with mild toxicity (mainly mild asthenia) Phlebitis at the infusion arm was common, concentration-dependent and avoidable by administration through a central vein catheter No haematological toxicity was observed
PK is characterised by dose-linearity, relatively prolonged body residence of the compound and extensive distribution. Potentially active plasma levels are reached from 2000 mcg/m²
An additional phase I study investigating an *iv* 3h infusion given every other week is ongoing. Starting dose 3000 mcg/m² q 2 weeks.

### Example 12

Phase I trial of aplidine given as a 1 hour intravenous weekly infusion in patients with advanced solid tumours and lymphoma

Adult patients with advanced disease, PS<3, and appropriate organ function are considered eligible; patients receive aplidine wk x 3/q4wks. 24 solid tumour patients have been entered: median (m) age 55 y, m ECOG=1, 15/24 patients treated with =>2 treatment cycles. Seven dose levels (DL) from 133 mcg/m²/wk to 2700 mcg/m²/wk have been assessed: 102 infusions are evaluable for toxicity (tox). No haematological tox has been reported, vomiting requiring prophylaxis was observed from 800 mcg/m²/wk. At 2700 mcg/m²/wk (4 pts), 1 had G3 hiperbilirrubinemia that has been considered to be dose limiting and therefore 2700 mcg/m²/wk DL is being expanded. All patients are being sampled for PK analysis (LC-ESI-MS/MS); kinetics are linear, the m Vss=308 L/m² CL is high m=0.60 L/ mon and the m elimination half life=14.2 h. Plasma levels >1 ng/ml 24 hours after infusion are reachable form 1800 mcg/m²/wk. Early hints of activity in gastric cancer have been noted (1 patient at 1200 mcg/m²). A patient with advanced renal cancer resistant to VBL-IFN has had an ongoing objective response (PR lung mets and SD in peritoneal disease) at 2700 mcg/m²/wk DL. Aplidine appears to be clinically feasible at pharmacologically appropriate dose levels.

### Example 13

A phase I and pharmacokinetic study of aplidine given as a 24-hour continuous infusion every other week (q2w) in patients with solid tumour and lymphoma

Aplidine was given to patients with solid tumour/ NHL as a 24 hr infusion/q2w to 35 patients (median: age=51/ECOG=1) with solid tumour (32 pts) or NHL (3 pts). 23/35 patients were previously exposed to ≥3 previous lines of chemotherapy. Nine dose levels (200-7000 µg/m²/w/q2w) and 65 cycles (120 infusions) were given. No haematiologic toxicity was reported. Toxicity consisted of G2-3 asthenia and emesis in 9-2 patients and 12-1 pts, respectively. G3 nausea/vomiting (≥5000 µg/m²) was efficiently treated then prevented with 4HT3-regimens. No cardiac toxicity was reported. At the dose of 5000 µg/m²/w/q2w, 2 patients experienced transient muscle cramps with reversible G3 CPK-MM elevations. Among 9 patients treated at 6000 µg/m²/w, 3 patients experienced an increase of CPK-MM and aldolase after the 3rd injection of aplidine. CPK elevations were G1-2 and not symptomatic in 2 patients but a G3 CPK elevation with G3 muscular pain and loss of muscle strength (DLT) was reported in 1 pt. This was reversible with no sequel. Muscle biopsy revealed no significant necrosis of mycocytes. Ultrastructural electron microscopy indicated no morphological mitochondria alteration but a lost of the thick myosin filaments. At 7000 µg/m²/q2w, 4 patients have been entered. Pharmacokinetic analysis (LC-ESI/MS/MS) showed that the AUC-increase is linear, with a large Vss=5391/m², a high clearance (332 ml/mm.m²), and a long terminal half life (15-35 h). Plasma concentrations 24 h after the end of infusion at doses ≥3000 µg/m²/q2w are comparable to efficient *in vitro* concentrations (<1 ng/ml). Activity was observed in NHL (1/3 pts), molecular thyroid (2/2 pts), renal (1 / 5 pts), and neuroendocrine cancer (1 pt). Mechanistic hypothesis and preventive strategies against muscle toxicity are under evaluation.

### Example 14 (Comparative example)

### Microarray assay

The human leukaemia cell line MOLT-4 was used for these experiments MOLT-4 cells were treated for 1 hour with 20 nM of aplidine. Total RNA was extracted at the end of treatment and 6 and 24 hours after recovery in drug-free medium.

5 µg of total RNA were retrotanscribed to cDNA in the presence of ³²P-dATP. Equal amounts of radioactive probes were hybridised to Atlas Human Cancer Microarrays (Clontech). After washing, filter were exposed and the results analysed using the Atlas Image software. Only a gene expression difference greater than 2 fold between treated and untreated cells were taken into account. RT-PCR and northern analysis have been performed to confirm the changes in gene expression found with the microarrays.

### Results

Aplidine treatment did cause significant changes in gene expression as early as 1 hour after treatment. After 6 and 24 hours of recovery in drug-free medium, the expression of an increased number of genes has been observed.

At the end of treatment the most significant changes were observed in the expression of the early response gene ETR, and in the VEGF-RI/flt-1 gene which were respectively increased and decreased by treatment.

ETR levels returned to normal level after 6 and 24 hours, while the levels of flt-1 further decreased by 6 and 24 hours.

Aplidine induced also an increase in the levels of B-RAF and Fms clearly observable 6 hours after recovery in drug-free medium.

The changes observed in these genes were confirmed by either RT-PCR or northern blot analysis.

From microarray analysis, differences in gene expression (not yet confirmed by RT-PCR) were observed for other genes such as PLK-1.

### Example 15

Phase I trial of aplidine, given as a 1h IV weekly infusion in patients with advanced solid tumours and non-Hodgkin lymphoma.

### Drug Administration

Aplidine was administered as a 1 hour weekly x3 every four weeks

### Patient Characteristics

| | | | |
|---|---|---|---|
| Number of patients | 30 | Tumour type | |
| Median age, years (ranges) | 53.5 (36-75) | Colorectal | 8 |
| ECOG performed status | | Lung | 5 |
| 0 | 2 | Gastric | 4 |
| 1 | 21 | Renal | 3 |
| 2 | 5 | Head and Neck | 3 |
| Prior radiotherapy | 10 | Melanoma | 2 |
| Prior chemotherapy (No. regimens) | | Ovary | 2 |
| 1 | 10 | Biliary tract | 1 |
| 2 | 10 | Oesophagus | 1 |
| ≥3 | 8 | Pancreas | 1 |

### Patient Accrual and Dose Escalation

| Dose level | Dose (mcg/m²/wk) | No. patients | No. cycles (range) |
|---|---|---|---|
| I | 133 | 3 | 5 (1-2) |
| II | 266 | 3 | 5(1-2) |
| III | 532 | 3 | 5 (1-2) |
| IV | 800 | 3 | 6 (2-2) |
| V | 1200 | 4 | 7 (1-2) |
| VI | 1800 | 4 | 7 (1-2) |
| VII | 2700 | 8 | 12 (1-2+) |
| VIII | 3600 | 2 | 3 (1-2+) |
| Total | | | |

| | | | |
|---|---|---|---|
| *one cycle definition: 3 consecutive weekly infusions with one week rest | | | |

### Worst Toxicities Per Patient

| Dose level (weekly x3) | 133 | 266 | 532 | 800 | 1200 | 1800 | 2700 | 3600 |
|---|---|---|---|---|---|---|---|---|
| No. patients | 3 | 3 | 3 | 3 | 4 | 4 | 8 | 2 |
| Nausea/vomiting G2 | - | - | - | 3 | - | 3 | 2 | - |
| Injection site reaction G2 | - | 1 | - | 1 | 1 | 1 | 3 | - |
| Asthenia G2(G3) | - | - | 1 | 1(1) | 3 | 2 | 1(1) | - |
| Myalgia G2(G3) | - | - | - | - | (1) | - | - | 1 |
| CPK elevation G 1 | - | - | - | 1 | - | - | - | 1 |
| Transaminitis G2(G3) | 1 | - | (1) | 1 | - | 1 | 1(4)⁽¹⁾ | - |
| BilirrubinG3 | - | - | - | - | - | - | 1(1)⁽²⁾ | - |
| Alk. Phos. G2 (G3) | - | (1) | - | - | - | 1 | (1)⁽²⁾ | - |
| Hypertension G1(G3) | (1) | - | - | 1 | - | - | - | - |

| | | | | | | | | |
|---|---|---|---|---|---|---|---|---|
| ⁽¹⁾one patient with Hepatitis C; 2 cases reversible by day 8 and 1 DLT ⁽²⁾DLT | | | | | | | | |

### Characterisation of the Dose Limiting Toxicity Reported

Pat #28 - Edinburgh with Oesophageal adenocarcinoma (no liver mets) treated at 2700 µg/m² weekly had delayed recovery of liver enzymes increase (G3 AST; G3 Bilirubin; G3 ALP) precluding the weekly administration of further doses.

### Hints of Activity

Pat # 16 - (Edinburgh) with Gastric adenocarcinoma primary resistant to FAM. Slight improvement in the lymph node masses around the lesser curve of the stomach, coeliac axis and pelvis with aplidine at 1200 µg/m² weekly (3600 µg/m²).

Pat #23 - (Barcelona) with Kidney carcinoma and pulmonary nodules as the main disease site, primary resistant to VBL + αIFN and to liposomal Doxorubicin. Partial remission in lung nodules and clinical improvement with resolution of dyspnea after 2 infusions of aplidine at 2700 µg/m² weekly (8100 µg/m²).

Pat #29 - (Barcelona) with kidney carcinoma. Clinical improvement after 3 infusion on the evaluation of a supraclavicular adenopathy.
Pending evaluation at 2nd cycle.

### Pharmacokinetic Data

Dose-linear PK up to the current dose level of 2700 µg/m² Important interpatient variability: coefficient of variation of CL, 33% Relatively high plasma CL: median (quartiles) 329 (288-407 mL/min/m²)
Intermediate t ½: median (quartiles) 15.8 (13.3-19.5) h.
Extensive distribution: median (quartiles) Vss, 345 (220-398) L/m²
Preliminary compartmental analysis: profiles are best fit by a first-order 3-compartment model with a very rapid initial phase (median half-life 0,04 h), followed by an intermediate phase (median half life 1.4 h) and a longer terminal phase (median half-life 20.7 h)

### Conclusions

This ongoing study indicates that aplidine given as a 1 hour infusion weekly with one week rest is clinically feasible up to a dose of 3600 mcg/m² every week. Bone marrow toxicity has not been reported. Emesis manageable by prophylactic antiemetics. Muscular toxicity consisting of muscular weakness and increased CK have been seen in one patient treated at 3600. Liver toxicity at the highest dose level investigated has been reported in several patients although was DLT in one patient. Pharmacokinetic information indicates that potentially therapeutic levels are achievable in plasma in patients treated from 1200 µg/m² onwards.

The feasibility of 3600 mcg/m² every week (dose level VIII) is being investigated

### Example 16

A phase I and pharmacokinetic study of aplidine, given as a 24h continuous infusion every other week in patients with solid tumours and non-Hodgkin lymphoma

**Patient Characteristics**

| | | | |
|---|---|---|---|
| Number of patients | 43 | Tumour type | |
| Median age, years (ranges) | 52(18-71) | Lung | 6 |
| ECOG performed status | | Colorectal | 8 |
| 0 | 19 | Kidney | 5 |
| 1 | 21 | Breast | 4 |
| 2 | 2 | Pancreas | 4 |
| Prior radiotherapy | 27 | Lymphoma | 3 |
| Prior chemotherapy (No. regimens) | | Ovary | 2 |
| 1 | 7 | Thyroid | 3 |
| 2 | 5 | Bone | 1 |
| ≥3 | 29 | Melanoma | 1 |
| | | Prostate | 1 |
| | | Uterus | 1 |
| | | Mesotheliorna | 1 |
| | | Gastric | 1 |
| | | Other | 2 |

**Patient Accrual and Dose Escalation**

| Dose level | Dose (mcg/m²/2wks) | No. patients | No. Cycles (range) |
|---|---|---|---|
| I | 200 | 3 | 5(1-3) |
| II | 400 | 3 | 6 (2-2) |
| III | 800 | 3 | 9 (2-4) |
| IV | 1600 | 6 | 11 (1-2) |
| V | 3200 | 3 | 5 (1-2) |
| VI | 4000 | 3 | 8 (2-4) |
| VII | 5000 | 3 | 6 (2-2) |
| VIII | 6000 | 12 | 26(1-6+) |
| IX | 7000 | 7 | 12(1-4+) |
| Total | | | |

| | | | |
|---|---|---|---|
| *cycle definition: 2bi-weekly infusions | | | |

**Worst Toxicities Per Patient.**

| Dose level | 200 | 400 | 800 | 1600 | 3200 | 4000 | 5000 | 6000 | 7000 |
|---|---|---|---|---|---|---|---|---|---|
| No. patients | 3 | 3 | 3 | 6 | 3 | 3 | 3 | 12 | 7 |
| Nausea/ | 2 | 1 | - | 4 | (1) | 2 | 1 | 2 | 1 |
| vomiting G2 (G3) | | | | | | | | | |
| Flushing G 1 | - | - | 1 | - | 1 | 1 | - | - | 1 |
| Asthenia G2 (G3) | - | 2 | - | 2(1) | 1 | 1 | (1) | 6 | 2 |
| Muscle cramps | - | - | 2 | 1 | 2 | 2 | 2 | 1 | - |
| G1+G2 | | | | | | | | | |
| Muscle pain G1 | - | - | - | - | (1) | 2 | 1 | 1(2) | 1 |
| (G2) | | | | | | | | | |
| Muscle weakness | - | - | - | - | | - | 1 | 1(1) | - |
| G1 (G2) | | | | | | | | | |
| CPK elevation | - | - | - | - | - | - | (1)[1] | 1(1) | 1 |
| (G2 (G3) [G4] | | | | | | | | | |
| Transaminitis | 1 | - | - | (1) | - | - | 1 | - | 1 |
| Transaminitis G2 (G3) | 1 | - | - | (1) | - | - | 1 | - | 1 |
| Hypertension G2 | - | 1 | - | - | - | - | - | - | - |
| Neutrophenia G4 | - | - | - | - | - | - | - | - | 1 |
| Pain central | - | - | - | - | - | 2 | - | - | - |
| catheter G2 | | | | | | | | | |

Characterisation of Muscular Toxicity (DLT)
Pat #27 - Male patient with medullary thyroid carcinoma treated at 6000 µg/m² weekly had symptomatic G3 CPK with G2 muscular pain. Toxicity recovered within 3 weeks after treatment discontinuation.

3 patients *(5000 and 6000 µg*/*m²*) experienced a minor elevations of CPKs (≥G2), consisting of CPK MM (muscle) increase with no significant elevation of CPK MB (heart). A parallel elevation of the aldolase level was observed. Signs of improvement by using Carnitine supplements as skeletal muscle protectors are being reported. Muscle biopsies were performed in 2 patients; E/M: partial disappearance of thick filaments of myosin.

### Pharmacokinetic Data

Aplidine appears to have a dose-linear PK profile (within the constraints imposed by the low sample size)
Relatively high plasma CL: median (quartiles) value 252 (192-415 mL/min/m²)
High interpatient CL variability (coefficient of variation of CL 62%)
Intermediate to long t ½ with a median (quartiles) value of 23.8 (15.7-35.0 h)
Wide distribution, median (quartiles) Vss of 413 (274-638 L/ m²)

Preliminary compartmental analysis: plasma profiles are best fit by a first-order 2-compartment model with a rapid initial (median half-life 0.64 h) and a longer terminal phase (median half-life 25.8 h)

Aplidine Mytotoxicity Relationship with Pharmacokinetics
Muscular toxicity has appeared only at high doses and exposures after 24 h infusion
Cmax values after 1 h infusion are already higher than those after 24 h infusion. Hence, a Cmax relationship may be ruled out
The AUC values in the patients with myotoxicity are high but not the maximum
It affected patients with high, sustained plasma concentrations of aplidine. The 3 patients with clear muscular toxicity had t ½ in excess of 44 h as compared to a median t ½ of 25.8 h after 24 h infusion

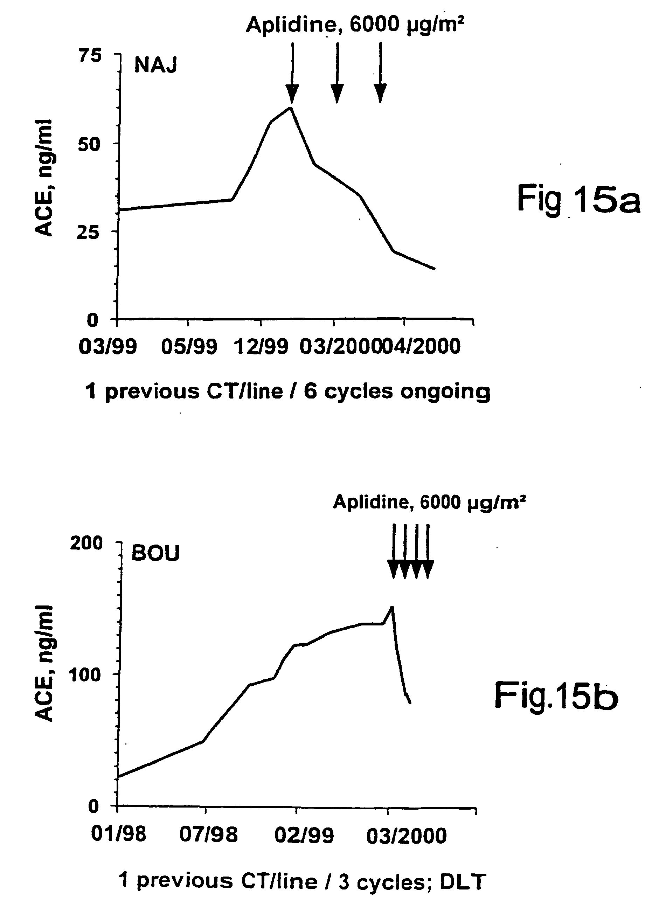

Figure 15a and 15b show the Activity in Medullary Thyroid Cancer. ACE Levels

### Conclusions

Drug induced muscular changes (expected to be the dose limiting toxicity), reported from dose level number III onwards (1800 mcg/m² to 5000 mcg/m²) is dose limiting toxicity at 6000 mcg/m² (1/9 pts)
Antitumour activity has been also noted in patients with NHL and renal carcinoma
The study is now investigating the feasibility of 6000-7000 mcg/m² every other week by using carnitine supplements as skeletal muscle protectors.

## Claims

1. The use of aplidine in the preparation of a pharmaceutical composition for a method of treating cancer in a patient which comprises administering aplidine by intravenous infusion in accordance with one of the following regimes:
a. 24 hour infusion weekly for three weeks, followed by one week rest, at a recommended dose of no more than 3750 mcg/m² /wk,
b. Biweekly 24 hour infusion at a recommended dose of no more than 7000 mcg/m²/2wk,
c. 1 hour infusion weekly for three weeks every 4 weeks at a recommended dose of no more, than 3600 mcg/m² /wk.

2. The use of aplidine according to claim 1 which comprises administering aplidine by intravenous infusion as a 24 hour infusion weekly for three weeks, followed by one week rest, at a recommended dose of no more than 3750 mcg/m²/wk.

3. The use of aplidine according to claim 1 which comprises administering aplidine by intravenous infusion as a biweekly 24 hour infusion at a recommended dose of no more 7000 mcg/m²/2wk.

4. The use of aplidine according to claim 3 which comprises administering aplidine by intravenous infusion as a biweekly 24 hour infusion at a dose of 5000 mcg/m²/2wk.

5. The use of aplidine according to claim 1 which comprises administering aplidine by intravenous infusion as a 1 hour infusion weekly for three weeks every 4 weeks at a recommended dose of no more than 3600 mcg/m²/wk.

6. The use of aplidine according to claim 5 which comprises administering aplidine by intravenous infusion as a 1 hour infusion weekly for three weeks every 4 weeks at a dose of 2700 mcg/m²/wk.

7. The use of any preceding claim, wherein the aplidine is administered as part of a combination therapy.

8. The use of any preceding claim, wherein the aplidine is administered in conjunction with a skeletal muscle protector.

9. The use of claim 8, wherein the skeletal muscle protector is carnitine.

10. The use of any preceding claim, wherein the patient has already received the standard treatment for his/her cancer disease and the tumor is refractory.

## Patentansprüche

1. Anwendung von Aplidin bei der Herstellung einer pharmazeutischen Zusammensetzung für ein Verfahren zur Behandlung von Krebs bei einem Patienten, die die Verabreichung von Aplidin durch intravenöse Infusion nach einem der folgenden Therapiepläne umfasst:
a. 24-stündige Infusion, wöchentlich für drei Wochen, gefolgt von einwöchigem Aussetzen, in einer empfohlenen Dosis von nicht mehr als 3750 µg/m²/Woche,
b. zweiwöchentliche 24-stündige Infusion in einer empfohlenen Dosis von nicht mehr als 7000 µg/m²/2 Wochen,
c. 1-stündige Infusion, wöchentlich für drei Wochen, alle 4 Wochen, in einer empfohlenen Dosis von nicht mehr als 3600 µg/m²/Woche.

2. Anwendung von Aplidin nach Anspruch 1, die die Verabreichung von Aplidin durch intravenöse Infusion als eine 24-stündige Infusion, wöchentlich für drei Wochen, gefolgt von einwöchigem Aussetzen, in einer empfohlenen Dosis von nicht mehr als 3750 µg/m²/Woche umfasst.

3. Anwendung von Aplidin nach Anspruch 1, die die Verabreichung von Aplidin durch intravenöse Infusion als eine zweiwöchentliche 24-stündige Infusion in einer empfohlenen Dosis von nicht mehr als 7000 µg/m²/2 Wochen umfasst.

4. Anwendung von Aplidin nach Anspruch 3, die die Verabreichung von Aplidin durch intravenöse Infusion als eine zweiwöchentliche 24-stündige Infusion in einer Dosis von 5000 µg/m²/2 Wochen umfasst.

5. Anwendung von Aplidin nach Anspruch 1, die die Verabreichung von Aplidin durch intravenöse Infusion als eine 1-stündige Infusion, wöchentlich für drei Wochen, alle 4 Wochen, in einer empfohlenen Dosis von nicht mehr 3600 µg/m²/Woche umfasst.

6. Anwendung von Aplidin nach Anspruch 5, die die Verabreichung von Aplidin durch intravenöse Infusion als eine 1-stündige Infusion, wöchentlich für drei Wochen, alle 4 Wochen, in einer Dosis von 2700 µg/m²/Woche umfasst.

7. Anwendung nach einem der vorangehenden Ansprüche, worin das Aplidin als Teil einer Kombinationstherapie verabreicht wird.

8. Anwendung nach einem der vorangehenden Ansprüche, worin das Aplidin zusammen mit einem Mittel zum Schutz der Skelettmuskulatur verabreicht wird.

9. Anwendung nach Anspruch 8, worin das Mittel zum Schutz der Skelettmuskulatur Carnitin darstellt.

10. Anwendung nach einem der vorangehenden Ansprüche, worin der/die Patient(in) bereits die Standardbehandlung für seine/ihre Krebserkrankung erhalten hat und der Tumor refraktär ist.

## Revendications

1. Utilisation d'aplidine dans la préparation d'une composition pharmaceutique pour une méthode de traitement du cancer chez un patient, qui comprend administrer l'aplidine par perfusion intraveineuse selon l'un des schémas suivants :
a. perfusion de 24 heures toutes les semaines pendant trois semaines, suivies par une semaine de repos, à une dose recommandée de pas plus de 3750 µg/m²/semaine,
b. perfusion de 24 heures toutes les deux semaines à une dose recommandée de pas plus de 7000 µg/m²/2 semaines,
c. perfusion d'une heure toutes les semaines pendant trois semaines, toutes les quatre semaines, à une dose recommandée de pas plus de 3600 µg/m²/semaine.

2. L'utilisation d'aplidine selon la revendication 1, qui comprend administrer l'aplidine par perfusion intraveineuse sous forme de perfusion de 24 heures toutes les semaines pendant trois semaines, suivies par une semaine de repos, à une dose recommandée de pas plus de 3750 µg/m²/semaine.

3. L'utilisation d'aplidine selon la revendication 1, qui comprend administrer l'aplidine par perfusion intraveineuse sous forme de perfusion de 24 heures toutes les deux semaines à une dose recommandée de pas plus de 7000 µg/m²/2 semaines.

4. L'utilisation d'aplidine selon la revendication 3, qui comprend administrer l'aplidine par perfusion intraveineuse sous forme de perfusion de 24 heures toutes les deux semaines à une dose de 5000 µg/m²/2 semaines.

5. L'utilisation d'aplidine selon la revendication 1, qui comprend administrer l'aplidine par perfusion intraveineuse sous forme de perfusion d'une heure toutes les semaines pendant trois semaines, toutes les quatre semaines, à une dose recommandée de pas plus de 3600 µg/m²/semaine.

6. L'utilisation d'aplidine selon la revendication 5, qui comprend administrer l'aplidine par perfusion intraveineuse sous forme de perfusion d'une heure toutes les semaines pendant trois semaines, toutes les quatre semaines, à une dose de 2700 µg/m²/semaine.

7. L'utilisation de l'une quelconque des revendications précédentes, où l'aplidine est administrée comme faisant partie d'une thérapie combinatoire.

8. L'utilisation de l'une quelconque des revendications précédentes, où l'aplidine est administrée conjointement avec un protecteur des muscles squelettiques.

9. L'utilisation de la revendication 8, où le protecteur des muscles squelettiques est la carnitine.

10. L'utilisation de l'une quelconque des revendications précédentes, où le patient a déjà reçu le traitement standard pour sa maladie cancéreuse et la tumeur est réfractaire.
